# EUROPEAN PATENT SPECIFICATION

(11) **EP 4 611 885 B1**
(45) Date of publication and mention of the grant of the patent: **14.10.2026**
(21) Application number: 24730444.7
(22) Date of filing: 29.04.2024
(51) Int. Cl.: A61N 5/06

(54) **DEVICE FOR THE TREATMENT OF OCULAR DISORDERS**
VORRICHTUNG ZUR BEHANDLUNG VON AUGENERKRANKUNGEN
DISPOSITIF POUR LE TRAITEMENT DE TROUBLES OCULAIRES

(30) Priority: 27.04.2023 IT 202300008307
(43) Date of publication of application: 10.09.2025
(73) Proprietor: Espansione Marketing S.p.A., 40050 Funo di Argelato (BO) (IT)
(72) Inventor: FIER, Alessandro, 40050 Argelato (BO) (IT)
(74) Representative: Manzella & Associati
(86) International application number: PCT/IT2024/050085
(87) International publication number: WO 2024/224431

(56) References cited:
- WO-A1-2023/283699
- US-A1- 2014 005 757
- US-A1- 2016 008 625
- US-A1- 2016 067 087
- US-A1- 2021 060 354
- US-A1- 2021 315 736
- US-A1- 2021 379 401

## Description

### Technical field

The present invention relates to a device for the treatment of ocular disorders, in particular for the treatment of disorders of the internal tissues of the eye.

### Prior art

The need to treat ocular disorders, in particular disorders of the internal tissues of the eye such as the retina, a thin membrane of nerve origin lining the internal surface of the eye and extending from the optic nerve to the pupillary orifice, has long been known.

Numerous retina's disorders include diabetic retinopathy, which causes damage to the retina's blood vessels, and retinal macular degeneration, a degenerative disease that affects retinal cells causing a loss of deep vision.

Both disorders, if not properly treated, can cause permanent damage to the retinal tissues, causing a degeneration of visual acuity and contrast sensitivity and, in the most severe cases, even the loss of visual function.

In particular, retinal macular degeneration is caused by age-dependent degeneration of the retinal cells, in fact, it tends to affect subjects who are over 60 years of age and it is estimated that about 50 million people worldwide suffer from it, being among the main causes of visual impairment in developed countries.

Age-dependent retinal macular degeneration is due to a progressive accumulation of mutations in mitochondrial DNA (mtDNA), which causes a decrease in the formation of ATP (Adenosine Triphosphate), a source of energy for cellular metabolism, and an increase in the production of reactive oxygen species, inducing oxidative stress, progressive inflammations, accumulation of extracellular debris with consequent marked cellular loss (approximately 30% of central rod photoreceptors is estimated to be lost by the age of 70).

Retinal macular degeneration occurs in two forms. In particular, there is a "dry", i.e. non-exudative, more widespread form (about 85% of cases), which causes modifications of the retinal pigment epithelium visible as punctiform dark spots and can cause drusen, i.e. accumulations of extracellular debris visible as yellow spots.

In addition, there is a "wet", exudative form, which occurs when the retina undergoes choroidal neovascularization, that is, a process of abnormal formation of blood vessels that can lead to a focal macular oedema or haemorrhage, leading to a localized detachment of the retinal epithelium and a rapid loss of visual function.

The treatments envisaged for these types of disorder consist of therapies with dietary supplements based on zinc oxide, copper, vitamin C, vitamin E and lutein, administration of drugs, in particular insulin for the diabetic forms of retinopathy and of intravitreal vascular endothelial growth factor antagonists for the forms of wet retinal macular degeneration. Retina's treatments through retinal laser treatments are also widespread, whose light beam, generated using the noble gas argon (argon laser), performs a thermal action, heating the area on which it is aimed, allowing it to be treated.

One problem with the treatments with dietary supplement administration is that they are able to provide benefits solely in the reduction of the risk of developing advanced forms of retina's disorders, but they are not able to effectively treat retinal cells, whereas treatments with intravitreal drug administration are not effective in treating all types of retinal degeneration. The administration of insulin, on the other hand, is able to treat diabetes at the systemic level, slowing down diabetic retinopathy, but it is not able to cure retinal cells.

A problem regarding treatments by retinal laser (argon laser), which are used both in the treatment of diabetic retinopathy and in the treatment of retinal macular degeneration, on the other hand, is that they are mainly aimed at destroying the retinal areas affected and damaged by these disorders, but they are not able to cure the retinal cells. Treatments by argon laser are also considered invasive treatments as they involve a direct intervention on the patient's eyes which, if not performed correctly, can lead to severe visual impairment of the patient and permanent damage to the eye tissues.

Therefore, the currently available treatments are not effective in treating disorders of the internal tissues of the eye, which are still treated or whose development is slowed down with difficulty, still severely affecting the quality of life of the patients who suffer from it.

A different type of device for treating retina's tissues is illustrated in patent US 2021/315736. The patent describes a wearable device comprising a first light source adapted to emit a light beam having a wavelength comprised in the near infrared and a second light source adapted to emit light at a wavelength comprised in an interval between 600 and 700 nm or between 550 and 650 nm. The device also comprises a diffuser adapted to diffuse the light of the first light beam and/or of the second light beam so that at least a portion of the first light beam and of the second light beam are directed through the retinal tissue of the patient when the patient is wearing the device.

Patent US 2016/067087 also illustrates a wearable device for directing a light-based therapy onto a patient's ocular tissues. The device comprises a first light source adapted to produce a light beam having a first wavelength comprised in an interval between 800 and 900 nm and at least a second light source adapted to produce a light beam having a second wavelength comprised in an interval between 600 and 700 nm or between 550 and 650 nm. The device further comprises a spatial light modulator adapted to receive light from the first and second light source and to modulate the light beam, which is directed to the patient's eye, so that at least a portion of the first light beam and a portion of the second light beam are directed towards the patient's eye when the patient is wearing the device.

A further example of device that uses light to treat ocular pathologies, such as diabetic retinopathy, wet type and dry type age-correlated macular degeneration (AMD), is illustrated in patent US 2016/008625.

Patent US 2021/060354 describes a prevention and treatment method for diabetic retinopathy and diabetic macular oedema, which provides for the administration to the eyes of a patient of an amount of light having a photobiomodulating effect, of wavelength in the infrared interval. In particular, the wavelengths envisaged for said treatment method are comprised around 670±50 nm and more in particular of 670±30 nm.

Patent US 2014/005757 describes a removable spare part adapted to be connected with a support device, which comprises a radiation source adapted to emit radiations in the direction of an area to be treated, in particular towards the eyes of a user, and a structure for positioning the radiation source in a predetermined position relative to the area to be treated. A wavelength comprised in the interval between 450 and 580 nm is mentioned for the treatment of diabetic retinopathy and macular degeneration.

Patent application WO 2023/283699 likewise describes a device for therapy by photobiomodulation.

Although several devices have been developed for the treatment of ocular disorders that use light at predetermined wavelengths, there is a need to identify improved solutions in terms of treatment efficacy.

### Disclosure

The task of the present invention is to solve the aforementioned problems, by devising a device for the treatment of ocular disorders capable of effectively treating disorders of the internal tissues of the eye.

Within the scope of this task, it is a further object of the present invention to provide a device for the treatment of ocular disorders capable of operating a non-invasive treatment of the disorders of the internal tissues of the eye.

A further object of the present invention is to provide a device that automatically performs the treatment of disorders of the internal tissues of the eye.

A further object of the invention is to provide a device for the treatment of ocular disorders of simple constructive and functional design, having reliable operation, versatile use, as well as relatively inexpensive cost.

The cited objects are achieved, according to the present invention, by the device for the treatment of ocular disorders according to Claim 1, by the computer program according to Claim 10 as well as by the readable memory according to Claim 18.

In particular, the device is adapted to treat structures located posteriorly to the eyeball.

The device for the treatment of ocular disorders comprises a mask, preferably having the form of an anthropomorphic mask.

A plurality of light sources are distributed on an internal surface of said mask.

Said light sources are of the type of light-emitting diodes, for simplicity's sake usually referred to by the acronym LED.

Advantageously, said light-emitting diodes are configured to emit electromagnetic radiations adapted to stimulate the cellular function of the retinal cells.

Said LEDs are distributed on said internal surface of said mask, turned towards the user's face in use. In particular, said LEDs are distributed so as to form at least one matrix in areas of said internal surface of said mask that face, in use, the ocular areas of the user.

Preferably, said LEDs are connected to appropriate electrical circuits that allow to manage the power supply and the correct operation of said diodes.

Preferably said LEDs are electrically connected to a control and/or power supply unit of the device.

Preferably, said control and/or power supply unit is arranged externally with respect to said mask and is connected to it, for example by means of an appropriate connection cable.

Said control and/or power supply unit comprises a control interface.

Preferably, said control interface is adapted to allow easy setting of commands relating to a treatment protocol of the internal tissues of the user's eye, in particular of the retina. For example, the interface can allow to start or stop a treatment protocol or to set the parameters that characterize such treatment, such as for example the duration of the treatment or the selection of the wavelengths emitted by said diodes to be activated.

Preferably the interface is provided with a screen.

Even more preferably said interface can consist of a touch screen or a screen associated with suitable command buttons.

Alternatively, it is possible to provide for said control interface to be associated with said mask.

Preferably said external control and/or power supply unit comprises an electronic computer and a memory readable by said electronic computer.

The memory readable by said electronic computer comprises instructions that, when executed by said electronic computer, cause said electronic computer to perform the following steps: receiving from an operator, through said control interface, commands relating to a protocol for treating the internal tissues of the user's eye; automatically selecting a plurality of said light-emitting diodes and automatically setting the wavelength and emission duration thereof; instructing the user to close the eyes by means of a warning signal; operating said plurality of said light-emitting diodes for a first time interval, in continuous mode, at a wavelength in an interval of ± 40 nm around 590 nm to emit electromagnetic radiations in the direction of the closed eyes of said user; instructing said user to open the eyes by means of a warning signal; operating said plurality of said light-emitting diodes for a second time interval, in pulsed mode, at a wavelength in an interval of ± 40 nm around 590 nm to emit electromagnetic radiations in the direction of the open eyes of said user; instructing the user to close the eyes by means of a warning signal; operating said plurality of said light-emitting diodes for said first time interval, in continuous mode, at a wavelength in an interval of ± 40 nm around 630 nm to emit electromagnetic radiations in the direction of the closed eyes of a user; instructing the user to open the eyes by means of a warning signal; operating said plurality of said light-emitting diodes for said second time interval, in pulsed mode, at a wavelength in an interval of ± 40 nm around 630 nm to emit electromagnetic radiations in the direction of the open eyes of said user.

Advantageously said warning signal is an acoustic signal and/or a vibration signal and/or a signal displayed on said control interface.

Preferably said signal displayed on said control interface is an image and/or a text message.

The device comprising the control unit provided with a memory comprising the instructions that allow to implement the steps described above has the effect of effectively treating ocular disorders, in particular disorders affecting the structures located posteriorly to the eyeball. Based on the experimental data, in particular the medical evidence deriving from a clinical test, it was found that the alternated use of pulsed light and light in continuous mode at the specified wavelengths, alternating open and closed eyes of the user, produces a surprising result in terms of treatment efficacy. In particular, the aforementioned steps performed by the device are effective for the treatment of retina's disorders such as, for example, retinal macular degeneration.

It can also be observed that the use of the device comprising a mask that is positioned at the face of a user gives the user him-/herself greater comfort during the treatment protocol. The mask gives more freedom in terms of positions that the user can assume, for example the user can lie down.

Said LEDs of said at least one matrix are configured to emit a beam of electromagnetic radiations at predetermined wavelengths to inhibit the cellular mechanisms involved in retinal macular degeneration and increase the mitochondrial metabolic activity of the retinal cells by photobiomodulation.

Advantageously, said LEDs are configured to emit a beam of electromagnetic radiations at a wavelength in an interval around 590 nm or equal to 590 nm (yellow light). The technical effect of these features is to inhibit the expression of the vascular endothelial growth factor (VEGF) and increase the expression of nitric oxide in retinal cells. Vascular endothelial growth factor (VEGF) is in fact a signalling protein that stimulates the formation of blood vessels that contribute to the development of the wet form of retinal macular degeneration, while nitric oxide reduces oxidative stress-mediated lesions in the cell and increases oxygen distribution.

Advantageously, said LEDs are also configured to emit a beam of electromagnetic radiations at a wavelength in an interval around 630 nm or equal to 630 nm (red light). It has been experimentally observed that radiations having said wavelength are adapted to promote electron transfer and oxygen binding of cytochrome C oxidase (CCO) by increasing the mitochondrial metabolic activity, understood as the production of ATP (Adenosine Triphosphate) molecules and oxygen, in retinal cells and inhibit inflammatory events by reducing cell death.

Said interval is preferably wide 40 nm around the indicated value, for yellow light and for red light, respectively.

In particular, said LEDs are of the high power type.

Preferably said mask is formed by a laminar body comprising said plurality of light sources.

Said mask is preferably associated with suitable support means adapted to keep, in use, said mask in a predetermined position in front of a user's face. In particular, said device is held by said support means at an optimal distance from the user's face.

Said LEDs are distributed on said mask at substantially the same distance from the eyes of the user. The distance is suitably calculated based on the characteristics of the wavelengths of the light beam emitted by said LEDs.

Preferably said LEDs are arranged, in use, at a distance from the area to be treated comprised between 1 cm and 4 cm.

It can be observed that this distance is determined based on the focus and absorption of the electromagnetic radiations, which is optimal within this interval.

Preferably said memory comprises instructions which, when executed by said electronic computer, cause said electronic computer to automatically select a plurality of light-emitting diodes and automatically set the wavelength and emission duration thereof to carry out a treatment of internal tissues of a user's eye, in particular of a user's retinal cells.

In particular, said memory may comprise instructions that, when executed by said electronic computer, cause said electronic computer, as a function of commands set by an operator through said control interface relating to a protocol for treating the internal tissues of the user's eye, to automatically select a plurality of said light-emitting diodes and automatically set the wavelength and emission duration thereof.

According to one aspect of the invention, it is possible to provide that said LED circuits are connected to a controller associated with said mask, adapted to manage the operation of said LEDs, and that said external unit is used solely for the function of electrically powering said LEDs. In this case, said control interface can be associated with said mask or with said external power supply unit.

Said laminar body of said mask is preferably made of polymeric material. Alternatively, it is possible to provide that said mask is made of other suitable materials such as skin, leather, fabric and/or paper derivatives.

Preferably said mask has the eye area full so as to house said LED matrix. More specifically, for each eye, an area suitable for housing a respective LED matrix is obtained in said mask. In this way, said LED matrices can be used for the treatment of pathologies affecting the internal tissues of the user's eye.

Preferably said matrices are sized so as to irradiate the eyeballs of the user also comprising periocular areas for diffuse irradiation.

Preferably said LEDs are regularly distributed on said mask at the eye area. In particular, in each row of said matrices, said LEDs are arranged at the same distance from one another.

Preferably said LEDs distributed on said mask at said eye area are inclined at a predetermined angle with respect to the surface of the mask.

Even more preferably said LEDs are inclined at an angle of 45°.

Preferably each LED matrix at one eye comprises a substantially central region, wherein the LEDs have a predefined size, and at least one pair of LEDs, arranged on opposite sides of the central region, having a larger size than the LED of the central region. The aforementioned inclination of the LEDs together with the described distribution were considered advantageous, based on experimental evidence, for treating the internal tissues of the eye.

Preferably, said mask can also have in other areas, for example at different parts of the face such as the forehead and/or cheeks, further LED matrices adapted to be used to carry out appropriate therapeutic treatments on the skin.

Preferably said matrices arranged at the eye area comprise a number of said LEDs per surface unit greater than the number of said LEDs per surface unit of said further matrices.

Preferably, said LEDs of said matrices arranged at the eye area have smaller sizes than those of said LEDs constituting said further matrices.

Preferably, the laminar body of said mask has a thickness suitable for the insertion of said LEDs and said relative circuits.

According to one aspect of the invention, said laminar body of said mask comprises a first external layer and a second internal layer, made integral to each other.

Preferably said LEDs and said relative circuits are housed between said first external layer and said second internal layer.

Preferably said second internal layer has a pair of openings at the eye area so as to expose said matrices of said LEDs towards the eyelids and the periocular areas.

Preferably, said openings are shaped like an oval so as to define respective full areas with a shape suitable for irradiating, through said matrix LEDs, the entire surface of the eyeballs together with the periocular areas.

A series of further openings, preferably shaped as strips, adapted to house said further LED matrices, can also be made on said second internal layer.

Preferably said strips have different lengths depending on the region of said mask in which they are made and are arranged sequentially one after the other along a longitudinal direction of said mask.

Advantageously, said LEDs distributed at different areas of the face other than the eye area may have different characteristics, for example they may be suitable for the emission of a red-coloured beam of light to stimulate the production of collagen, or a blue-coloured beam to counteract bacterial acne or a yellow-coloured beam to stimulate the lymphatic system and the nervous system, or even an infrared beam. Preferably LEDs having different characteristics are combined in said same mask to perform mixed treatments.

It is possible to provide that the surfaces of said LEDs turned towards the skin to be treated are covered with an appropriate filter adapted to eliminate any potentially dangerous frequencies present in the emission spectrum of said LEDs.

Preferably said device comprises an image acquisition device adapted to acquire images of the eyes of a user and/or movement sensor means adapted to detect whether the user has open or closed eyes. In particular, said image acquisition device is used to check whether the user has open eyes or closed eyes.

Preferably said device comprises an acoustic signal transmitter adapted to transmit an acoustic signal to the user, to instruct him/her to open or close the eyes.

Advantageously said acoustic signal transmitter is adapted to transmit a first acoustic signal and a second acoustic signal, wherein the first acoustic signal is different from the second acoustic signal. By way of example, the first acoustic signal may differ from the second acoustic signal in the emission duration.

Preferably the first acoustic signal indicates to the user to close the eyes and the second acoustic signal indicates to the user to open the eyes.

The present invention also concerns a computer program implemented by the device for the treatment of the internal tissues of the eye, in particular of the retina's tissues.

The computer program comprises instructions that cause the device to perform the step of receiving from an operator, through said control interface, commands relating to a protocol for treating the internal tissues of the user's eye.

Preferably, said step of receiving from an operator, through said control interface, commands relating to a protocol for treating the internal tissues of the user's eye provides for receiving an activation command to initiate a treatment protocol. In particular, the operator can press a start button or select an appropriate icon on the interface to start this protocol.

Said program then provides for automatically selecting a plurality of said LEDs and automatically setting the wavelength and emission duration thereof.

The program provides for instructing the user to close the eyes by means of a warning signal.

The program provides for operating said plurality of LEDs for a first time interval, in continuous mode, at a wavelength in an interval of ± 40 nm around 590 nm to emit electromagnetic radiations in the direction of a user's closed eyes.

Subsequently, the program provides for instructing the user to open the eyes by means of a warning signal.

The program then provides for operating said plurality of LEDs for a second time interval, in pulsed mode, at a wavelength in an interval of ± 40 nm around 590 nm to emit electromagnetic radiations in the direction of a user's open eyes.

The next step provides for instructing the user to close the eyes, by means of a warning signal, and for subsequently operating said plurality of LEDs for said first time interval, in continuous mode, at a wavelength in an interval of ± 40 nm around 630 nm to emit electromagnetic radiations in the direction of a user's closed eyes.

Finally, the program provides for instructing the user to open the eyes by means of a warning signal.

The program then provides for operating said plurality of said LEDs for said second time interval, in pulsed mode, at a wavelength in an interval of ± 40 nm around 630 nm to emit electromagnetic radiations in the direction of a user's open eyes.

It has been experimentally observed that the aforementioned steps performed by the device are effective for the treatment of retina's disorders such as, for example, retinal macular degeneration. More specifically, the aforementioned steps can lead to a reduction of oxidative stress in the retinal cells and inhibition of the inflammation mediators with a decrease in the progressive inflammatory episodes that generate accumulations of extracellular debris and retinal cell loss, improving visual acuity and contrast sensitivity and decreasing the volume of drusen and macules in patients with degenerative diseases such as senile retinal macular degeneration. These effects are due to the combination of the treatment times and treatment modalities, i.e. with the patient having open or closed eyes, together with the wavelengths used.

As already mentioned, radiations having a wavelength in the indicated interval or substantially equal to 590 nm are adapted to inhibit the expression of the vascular endothelial growth factor (VEGF) and increase the expression of nitric oxide in the retinal cells while radiations having a wavelength of 630 nm are adapted to promote electron transfer and oxygen binding of cytochrome C oxidase (CCO) by increasing mitochondrial metabolic activity, understood as the production of ATP (Adenosine Triphosphate) molecules and oxygen, in the retinal cells and inhibit inflammatory events by reducing cell death.

Experimental results show that this program can also be effectively used in the treatment of amblyopia, retinitis pigmentosa and inflammation of the cornea, where a decrease in inflammatory cytokine levels and a restoration of irregularities caused by cell damage in corneal and retinal cells have been experimentally verified. It also improves the condition of the patients suffering from *dry eye disease* (DED) by increasing tear volume.

The program is also useful for wound healing following eye trauma or surgery.

It can also be observed that the program, once the commands on a treatment protocol have been received from an operator, allows the treatment to be administered automatically and easily to a patient.

Preferably said warning signal is an acoustic signal and/or a vibration signal and/or a signal displayed on said control interface.

Preferably said signal displayed on said control interface is an image and/or a text message.

According to one aspect of the invention, the program comprises the step of checking whether the eyes of said user are closed or open before the steps of operating the predetermined wavelength LEDs.

Preferably, said step of checking whether the eyes of said user are closed or open is carried out by said operator. Alternatively, said step of checking whether the eyes of said user are closed or open is performed by means of an image acquisition device, adapted to acquire at least one image of the eyes of the user, and/or by motion sensor means.

Advantageously, said first time interval is greater than said second time interval.

Preferably, said first time interval is greater than 5 minutes and said second time interval is less than 2 minutes.

Even more preferably, said first time interval is equal to 6 minutes, or around ± 1 minute with the duration of 6 minutes.

Even more preferably, said second time interval is equal to 1 minute, or around ± 15 seconds with the duration of 1 minute.

Advantageously, both eyes of the patient are simultaneously subjected to treatment with electromagnetic radiations by means of said device.

Preferably the aforementioned steps performed by said device constitute a treatment session.

Advantageously, the program comprises the further instruction to repeat said steps constituting a treatment session for at least one further treatment session, preferably for further 6-7 said sessions, thus defining a treatment cycle.

Preferably, the program comprises the further instruction to set a time interval between a session and a further session comprised between 3 and 4 days.

Preferably said program comprises the instruction to set a repetition of said treatment cycle, by operating said LEDs, after a period comprised between 6 and 9 months from the first execution, for a total of a predetermined number of sessions, preferably comprised between 4 and 7, preferably equal to 6 sessions.

In the event that a mixed treatment is carried out, for example a treatment of the eye area together with a therapeutic treatment of other areas of the face, said further LED matrices arranged at the affected regions are also activated.

In the case of mixed treatment, the operator proceeds, through said interface of said control and/or power supply unit, with selecting said further LEDs of interest and with entering the values of the parameters suitable for the type of treatment, for example the duration and the emission power.

### Brief description of the drawings

The details of the invention will become more apparent from the detailed description of a preferred embodiment of the device for the treatment of ocular disorders according to the invention, illustrated by way of example in the accompanying drawings, in which:
figure 1 shows a front perspective view of a component of the device;
figure 2 shows a front anterior view of an embodiment of a component of the device;
figure 3 shows a front view of an embodiment of a component of the device;
figure 4 shows a rear front view of a component of the device;
figure 5 shows a front view of a different embodiment of the component of the device illustrated in figure 2;
figure 6 shows a front view of a different embodiment of the component of the device illustrated in figure 4;
figure 7 shows a front view of a different embodiment of the component of the device illustrated in figure 3;
figure 8 shows a front perspective view of a control unit of the device;
figure 9 shows an image of a user wearing a component of the device according to the present invention;
figures 10a, 10b, 10c, 10d show successive steps of a treatment protocol implemented by an electronic computer of the device;
figures 11-13 show images of the retina of a same eye of a subject before and after a treatment employing the device according to the present invention;
figure 14a shows images of a subject's retina acquired by SD-OCT (Spectral-Domain Optical Coherence Tomography) technique under a condition of reference;
figure 14b shows images of the retina of the same subject as figure 14a, acquired by SD-OCT technique, one month after a treatment carried out by means of the device according to the present invention;
figure 14c shows images of the retina of the same subject of figures 14a and 14b, acquired by SD-OCT technique, three months after a treatment carried out by means of the device according to the present invention;
figures 15a and 15b show respectively an image of the ocular fundus of the same subject of figures 14a-14c, obtained by autofluorescence examination (FAF Fundus Autofluorence) in a condition of reference, and an image of the ocular fundus of the same subject after three months from a treatment carried out by means of the device;
figures 16a and 16b respectively show a map of the sensitivity of the retina of the same subject of figures 15a-15b, obtained with microperimetry, in a condition of reference and a map of the sensitivity of the retina of the same subject three months after treatment with the device.

### Description of embodiments of the invention

With particular reference to these figures, the device for the treatment of ocular disorders, in particular for the treatment of disorders of the internal tissues of the eye according to the present invention is indicated as a whole with 1.

The device 1 comprises a mask 2, preferably having the form of an anthropomorphic mask. The mask 2 is formed by a laminar body comprising a plurality of light sources 3 of the type of light-emitting diodes, for simplicity's sake usually indicated by the acronym LED, arranged on an internal surface of the mask 2.

In particular, the LEDs 3 are of the high-power type.

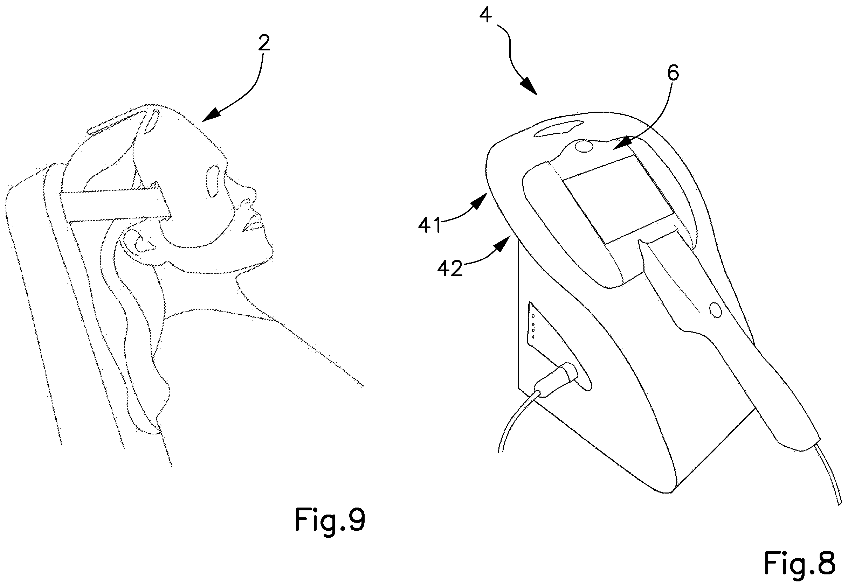

The mask 2 is preferably associated with suitable support means, visible in figure 9, adapted to hold, in use, the same mask in a predetermined position in front of the user's face.

In particular, the device 1 is held by the support means at an optimal distance from the user's face so that the distance between the LEDs 3 and the eyes of the user is kept in an interval comprised between 1 cm and 4 cm. This distance is determined based on the focus and absorption of the electromagnetic radiations, which is optimal within this interval.

The LEDs 3 are connected to appropriate electrical circuits, known per se and therefore not represented, which allow to manage the power supply and the correct operation of the diodes.

The LED circuits 3 are electrically connected to a control and/or power supply unit 4 of the device. The control and/or power supply unit 4 is arranged externally with respect to the mask 2 and is connected to it, for example by means of an appropriate connection cable (not visible in the figures).

The control unit 4 comprises a control interface 6 adapted to allow easy setting of the commands relating to a treatment protocol of the internal tissues of the eye, in particular of the retina, or of the parameters that characterize such treatment, such as for example the duration of the treatment or the selection of the wavelengths emitted by the diodes to be activated alternately depending on the step of the current treatment protocol, as will be better explained below. The control interface 6 also allows the possibility of displaying appropriate signals that help perform the treatment protocol, as will be better explained below.

The control interface 6 is provided with a screen. In particular, the interface 6 can consist of a touch screen or a screen associated with suitable control buttons.

Alternatively, it is possible to provide for the control interface that sets the treatment parameters to be associated with the mask 2.

The external control and/or power supply unit 4 comprises an electronic computer 41 and a memory 42 readable by an electronic computer.

The memory 42 comprises instructions which, when executed by the electronic computer 41, cause the electronic computer 41 to automatically select a plurality of light-emitting diodes 3 and automatically set the wavelength and emission duration thereof to carry out a treatment of a user's retinal cells.

In particular, the memory 42 may comprise instructions that, when executed by the electronic computer 41, cause the electronic computer 41, as a function of commands set by an operator through the control interface 6 relating to a protocol for treating the internal tissues of the user's eye, to automatically select a plurality of said light-emitting diodes 3 and automatically set the wavelength and emission duration thereof.

The memory 42 also comprises instructions that, when executed by the electronic computer 41, cause the electronic computer 41 to instruct a user to open or close the eyes. The signalling takes place by sending a vibration signal and/or an acoustic signal and/or by means of a warning signal displayed on the interface 6.

The warning signal may be an image appearing on the interface 6, in particular on the screen, indicating to open or close the eyes. The warning signal can be, alternatively or together with the image, a text message, always displayed on the interface 6, which performs the same function of informing that it is necessary to open or close the eyes.

In a different embodiment, it is possible to provide that the circuits of the LEDs 3 are connected to a controller associated with the mask 2, adapted to manage the operation of the LEDs 3, and that the external unit 4 is used only for the function of electrically powering the LEDs 3. In this case, the control interface 6 can be associated with the mask 2 or with the external power supply unit 4.

The laminar body of the mask 2 has a thickness suitable for the insertion of the LEDs 3 and the relative circuits. The LEDs are distributed on the internal surface of the mask 2, turned towards the user's face in use. In particular, the LEDs 3 are distributed so as to form at least one matrix 30 in the areas of the internal surface of the mask 2 facing, in use, the eyelids and the periocular areas of the user.

The matrix 30 preferably comprises LEDs 3 emitting light at a wavelength in an interval around 590 nm or equal to 590 nm (yellow light) and LEDs 3 emitting light at a wavelength in an interval around 630 nm or equal to 630 nm (red light). Advantageously, the LEDs 3 emitting yellow light and the LEDs 3 emitting red light can be activated alternatively, in use, depending on the step of the treatment protocol being executed.

The aforesaid interval is preferably wide 40 nm around the indicated value, for yellow light and for red light, respectively.

The laminar body of the mask 2 is preferably made of polymeric material. Alternatively, it is possible to provide that the mask 2 is made of other suitable materials such as skin, leather, fabric and/or paper derivatives.

The mask 2 has a full eye area so as to house the aforementioned matrix 30 of LEDs 3. More specifically, for each eye, an area suitable for housing a respective matrix 30 of LEDs 3 is obtained in the mask 2. In this way, the matrices 30 of LEDs 3 can be used for the treatment of pathologies affecting the internal tissues of the user's eye. More specifically, the matrices 30 are sized so as to irradiate the eyeballs of the user also comprising the periocular areas for diffuse irradiation.

Preferably the LEDs 3 are regularly distributed on the mask 2 at the eye area. In particular, in each row of the matrices 30, the LEDs 3 are arranged at the same distance from each other.

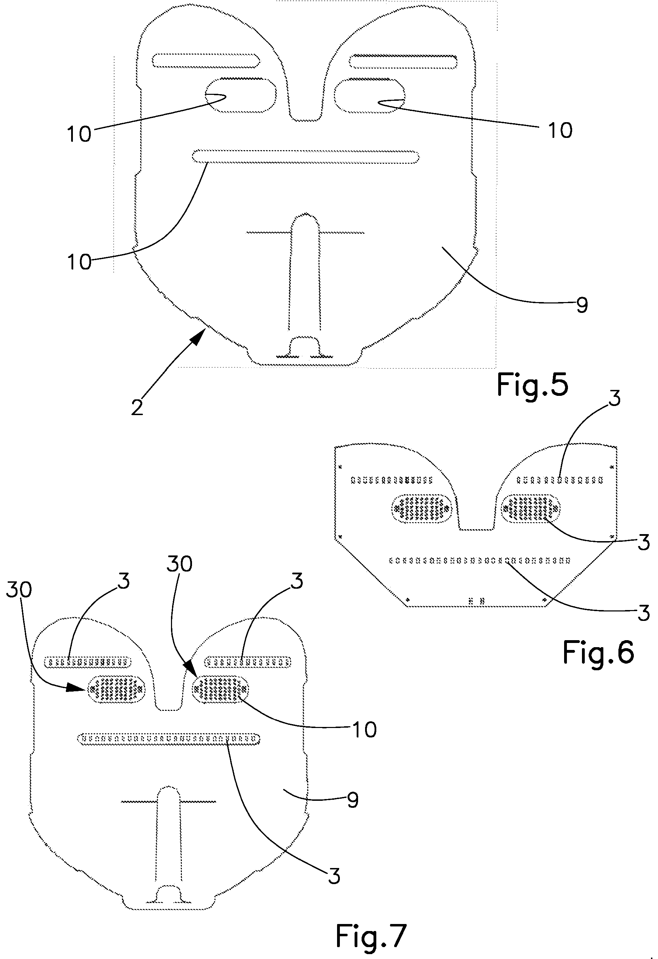

According to the embodiment of the mask 2 illustrated in figures 5-7, the matrices 30 arranged at the eye area each comprise a plurality of LEDs 3 which are inclined at a predetermined angle with respect to the surface of the mask. Preferably the LEDs 3 are inclined at an angle of 45°.

Each matrix 30 arranged at an eye comprises a substantially central region in which the LEDs 3 have a predefined size and at least one pair of LEDs 3, arranged on opposite sides of the central region, which have a larger size than the central LEDs 3.

The configuration of the mask according to figures 5-7 has been evaluated, based on clinical evidence, as particularly advantageous for administering treatments to the internal tissues of the eye.

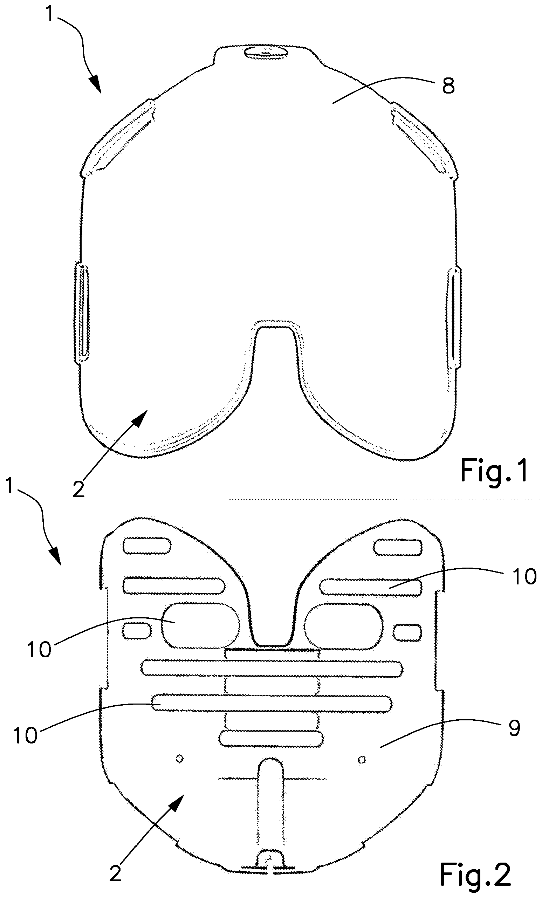

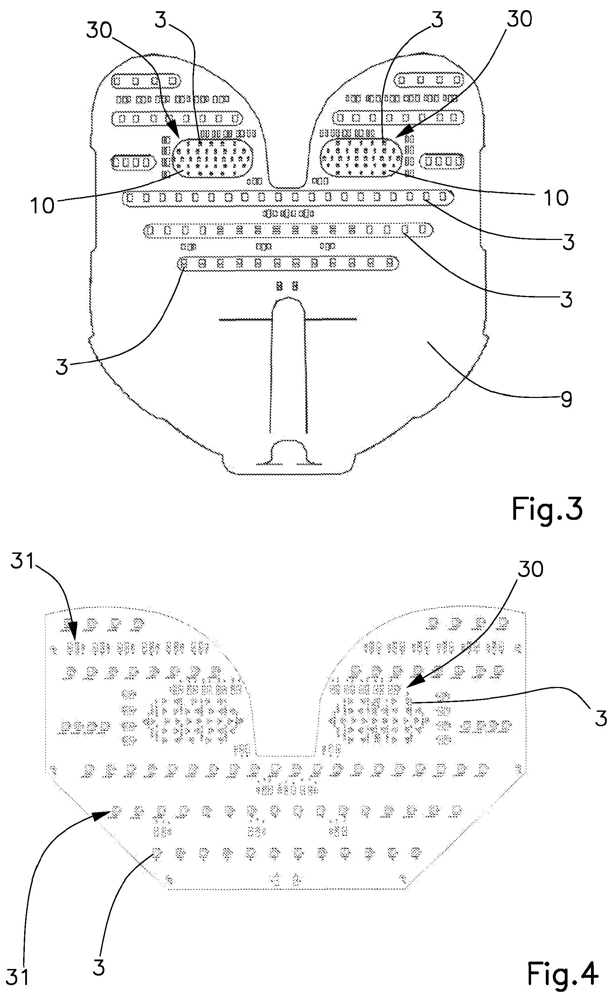

The mask 2 can also have in other areas, at for example different parts of the face such as the forehead and/or cheeks, further matrices 31 of LEDs 3 adapted to be used to carry out appropriate therapeutic treatments on the skin (see figures 1-4).

The matrices 30 arranged at the eye area comprise a number of LEDs 3 per surface unit greater than the number of LEDs 3 per surface unit of the further matrices 31.

Preferably, the LEDs 3 of the matrices 30 have smaller sizes than those of the LEDs 3 that make up the further matrices 31.

According to a preferred embodiment, the laminar body of the mask 2 consists of a first external layer 8 and of a second internal layer 9, made integral with each other.

The LEDs 3 and the relative circuits are housed between the first external layer 8 and the second internal layer 9. The second internal layer 9 has a pair of openings 10 at the eye area so as to expose the matrices 30 of the LEDs 3 towards the eyelids and periocular areas.

Preferably, the openings 10 are shaped like an oval so as to define respective full areas with a shape suitable for irradiating, through the LEDs 3 of the matrices 30, the entire surface of the eyeballs together with the periocular areas.

A series of further openings 10, preferably formed in strips, which are adapted to house the further matrices 31 of LEDs 3 can also be made on the second internal layer 9. The strips have different lengths depending on the region of the mask in which they are made and are arranged sequentially one after the other along a longitudinal direction of the mask.

The LEDs 3 are distributed on the mask 2 at substantially the same distance from the user's eyelids. The distance is appropriately calculated based on the wavelength characteristics of the light beam emitted by the LEDs 3.

Preferably, the LEDs 3 are arranged, in use, at a distance from the area to be treated comprised between 1 cm and 4 cm.

The LEDs 3 distributed at different areas of the face other than the eye area, i.e. the LEDs 3 that make up the matrices 31, may exhibit different characteristics, for example they may be suitable for emitting a red-coloured beam of light to stimulate the production of collagen, or a blue-coloured beam to counteract bacterial acne or a yellow-coloured beam to stimulate the lymphatic system and the nervous system, or even an infrared beam. Preferably LEDs 3 having different characteristics are combined in the same mask 2 to perform mixed treatments.

The LEDs 3 of the matrices 30 at the eye area are configured to emit a beam of electromagnetic radiations having predetermined wavelengths to inhibit the cellular mechanisms involved in retinal macular degeneration and increase the mitochondrial metabolic activity of the retinal cells by photobiomodulation.

It is possible to provide that the surfaces of the LEDs 3 turned towards the skin to be treated are covered with an appropriate filter adapted to eliminate any potentially dangerous frequencies present in the emission spectrum of the LEDs 3.

The present invention also concerns a computer program implemented by the device for the treatment of the internal tissues of the eye, in particular of retina's tissues, in which commands are set by the operator through the control interface 6, which provides for the exposure of the retinal cells to a predetermined amount of electromagnetic radiations so as to cause changes at the molecular level in the retinal cells, useful for the treatment of retina's disorders such as retinal macular degeneration.

In fact, it has been experimentally observed that radiations having a wavelength in the indicated interval or substantially equal to 590 nm are adapted to inhibit the expression of the vascular endothelial growth factor (VEGF) and increase the expression of nitric oxide in retinal cells.

Vascular endothelial growth factor (VEGF) is a signalling protein that stimulates the formation of blood vessels which contribute to developing the wet form of retinal macular degeneration, while nitric oxide reduces oxidative stress-mediated lesions in the cell and increases oxygen distribution

In addition, it has been experimentally observed that radiations having a wavelength of 630 nm are adapted to promote electron transfer and oxygen binding of cytochrome C oxidase (CCO) by increasing mitochondrial metabolic activity, understood as the production of ATP (Adenosine Triphosphate) molecules and oxygen, in retinal cells and inhibit inflammatory events by reducing cell death.

Such a program implemented by the device can therefore lead to a reduction of the oxidative stress in the retinal cells and to the inhibition of the inflammation mediators with a decrease in the progressive inflammatory episodes that generate accumulations of extracellular debris and retinal cell loss, improving visual acuity and contrast sensitivity and decreasing the volume of drusen and macules in patients with degenerative diseases such as senile retinal macular degeneration.

Such a program can also be effectively used in the treatment of amblyopia, retinitis pigmentosa and corneal inflammation, where a decrease in inflammatory cytokine levels and a restoration of the irregularities caused by cell damage in corneal and retinal cells has been experimentally verified. It also improves the condition of the patients suffering from *dry eye disease* (DED) by increasing tear volume.

It is also useful for wound healing following eye trauma or surgery.

Preferably the device 1 is associated with an image acquisition device, not illustrated in the figures. The image acquisition device may be, for example, a camera or a video camera, and is used to acquire images of the eyes of the user in order to check, during treatment, whether the eyes are open or closed. As an alternative to or in conjunction with the image acquisition device, the device 1 comprises motion sensor means adapted to detect whether the eyes of the user are closed or open.

Finally, the device 1 may also comprise an acoustic signal transmitter, also not represented in the figures, adapted to send an acoustic signal, during treatment, to signal the need to open or close the eyes.

The operation of the device for the treatment of the internal tissues of the eye is easily understandable from the foregoing description.

First, the mask 2 is placed in front of the user's face using the suitable support means (see figure 9).

An operator sets through the interface 6 of the control and/or power supply unit 4 an activation command relating to a protocol for treating the internal tissues of a user's eye. Essentially, an operator can press a start button or select an appropriate icon on the interface to start the treatment protocol.

The program executed by the electronic computer 41 selects the LEDs 3 arranged at the eye area, and automatically sets the wavelength and the emission duration of the LEDs 3 suitable for the treatment to be carried out.

The user is then subjected to the treatment for a predetermined time interval that corresponds to the emission duration Δt.

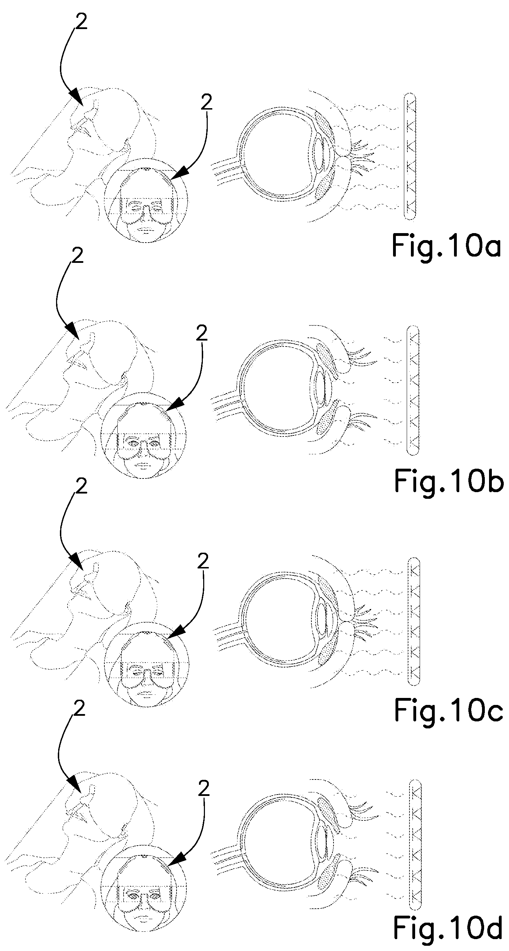

In particular, the user is instructed to close the eyes and the LEDs 3 are then operated for a first time interval Δt₁, in continuous mode, at a wavelength in an interval of ± 40 nm around 590 nm, preferably substantially equal to 590 nm, to emit electromagnetic radiations in the direction of the closed eyes of the user (see figure 10a).

Subsequently, the user is instructed to open the eyes and the LEDs 3 are operated for a second set time interval Δt₂, in pulsed mode, at a wavelength in an interval of ± 40 nm around 590 nm, preferably substantially equal to 590 nm to emit electromagnetic radiations in the direction of the user's open eyes (see figure 10b).

Additionally, the user is instructed to close the eyes and the LEDs 3 are operated for the first set time interval Δt₁, in continuous mode, at a wavelength in an interval of ± 40 nm around 630 nm, preferably substantially equal to 630 nm to emit electromagnetic radiations in the direction of the closed eyes of the user (see figure 10c).

Subsequently, the user is instructed to open the eyes and the LEDs 3 are operated for the second set time interval Δt₂, in pulsed mode, at a wavelength in an interval of ± 40 nm around 630 nm, preferably substantially equal to 630 nm to emit electromagnetic radiations in the direction of the open eyes of a user (see figure 10d).

The opening or closing of the eyes is instructed to the user by sending an acoustic signal and/or a vibration signal and/or a warning signal displayed on the control interface.

Preferably, the program provides for the further step of checking whether the eyes of the user are closed or open by means of the image acquisition device, adapted to acquire at least one image of the eyes of the user, and/or by the motion sensor means. Alternatively, the check can be carried out by the operator in charge of the treatment control.

Advantageously, the first time interval Δt₁ is greater than the second time interval Δt₂.

Preferably, the first time interval Δt₁ is greater than 5 minutes and the second time interval Δt₂ is less than 2 minutes.

For example, the first time interval Δt₁ is 6 minutes, or around ± 1 minute with the duration of 6 minutes.

For example, the second time interval Δt₂ is equal to 1 minute, or around ± 15 seconds with the duration of 1 minute.

Advantageously, both eyes of the patient are simultaneously subjected to treatment with electromagnetic radiations by means of the device 1.

The above treatment steps are performed by the computer program using LEDs 3 on the eyes of a user in a treatment session. It is preferable to repeat the treatment up to 7 or 8 sessions, at certain time intervals, preferably every 3 or 4 days, to constitute a cycle of sessions.

Preferably the cycle of treatment sessions is again performed by the computer program by means of the LEDs 3 on the eyes of a user after a period comprised between 6 and 9 months from the first execution. The number of sessions for this next treatment cycle is preferably comprised between 4 and 7, preferably it is equal to 6 sessions.

It is preferable to perform a check of the health status of the cells of the internal tissues of the eye at predetermined time distances, for example after 1 month, 2 months and 4 months from the end of the last treatment cycle.

A randomized controlled multicentre clinical trial has been performed on patients presenting with AMD (Age-related Macular Degeneration) grades 2 and 3 according to the AREDS classification. Devices according to the present invention have been used in the clinical test, wherein the memory of each device comprises instructions that, when executed by the electronic computer 41 cause the electronic computer 41 to perform the steps described above. The aforementioned steps involve the administration of electromagnetic radiations at specific wavelengths for predetermined time intervals towards the closed or open eyes of each patient.

The objective of the set up clinical trial was to check whether the device implementing the aforementioned steps has the effect of slowing down the progression of the disease in advanced stages. The results of the clinical test carried out were indicative of the efficacy of the treatment administered by means of the device.

By way of example, below are two cases taken from the clinical test carried out.

### Case 1

The treated subject is a 68-year-old woman with grade 3 age-related macular degeneration (ADM), according to the AREDS classification.

The woman's best corrected visual acuity before treatment was quantified with 50 letters on the Early Treatment Diabetic Retinopathy Study (ETDRS) table.

The woman underwent a treatment, using the device that implements the described program, which involves twice-weekly sessions lasting four weeks.

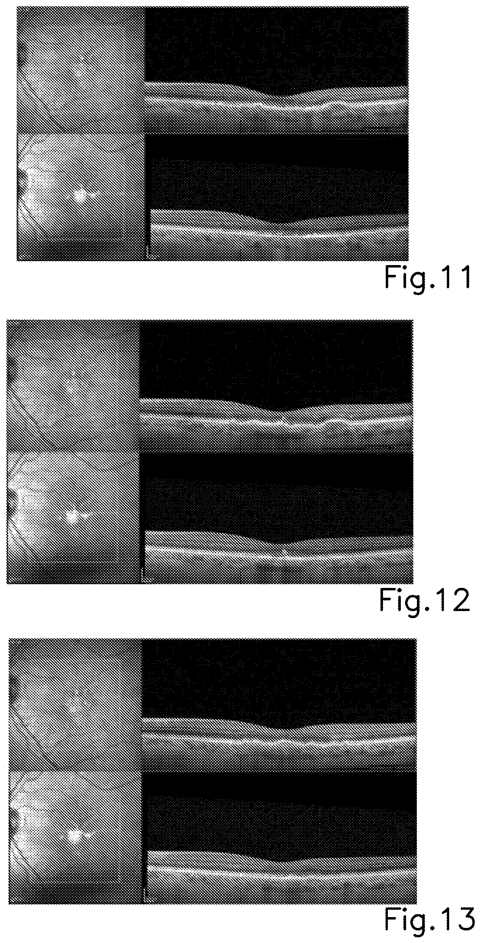

An assessment of the woman's health status following one-month treatment was performed and, in particular, the best corrected visual acuity of 55 ETDRS letters was measured. In addition, a decrease in drusen volume in the macular region was observed by acquiring a series of images of the woman's retina, as shown in figures 11-13.

### Case 2

The treated subject is a 55-year-old man with non-vascular age-related macular degeneration. The best corrected visual acuity measured before treatment was 25 letters on the ETDRS table. The SD-OCT (Spectral-Domain Optical Coherence Tomography) technique had highlighted a substantial detachment of the pigment epithelium together with a subretinal fluid at its apex.

The man underwent a treatment, by means of the device that implements the described steps. Treatment comprised one session per week for the duration of four weeks. Following the first month of treatment, the man underwent one session every two weeks for the duration of two months.

One month after the end of the treatment, complete resorption of the subretinal fluid and a reduction in the detachment of the pigment epithelium without residual retinal atrophy were observed. The best corrected visual acuity was measured at 60 ETDRS letters.

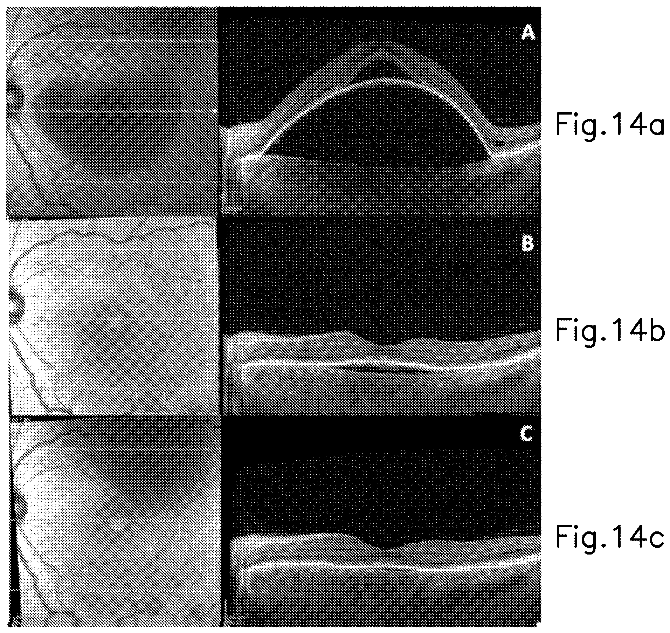

The improvement of the health condition of the man is also documented by the images acquired at different time intervals (see figures from 14a to 16b). In particular, figure 14a shows images of the man's retina, acquired with the SD-OCT technique, which represent a condition of reference of the man before treatment. Images in the condition of reference show pronounced pigment epithelial detachment (PED) with subretinal fluid.

Figure 14b shows, on the other hand, images of the man's retina, always acquired with the SD-OCT technique, one month after treatment. The images highlight a flattening of the detachment of the pigment epithelium together with hyper/hypo-reflective material.

The images of figure 14c were acquired with the SD-OCT technique three months after treatment and show a further flattening of the detachment of the pigment epithelium.

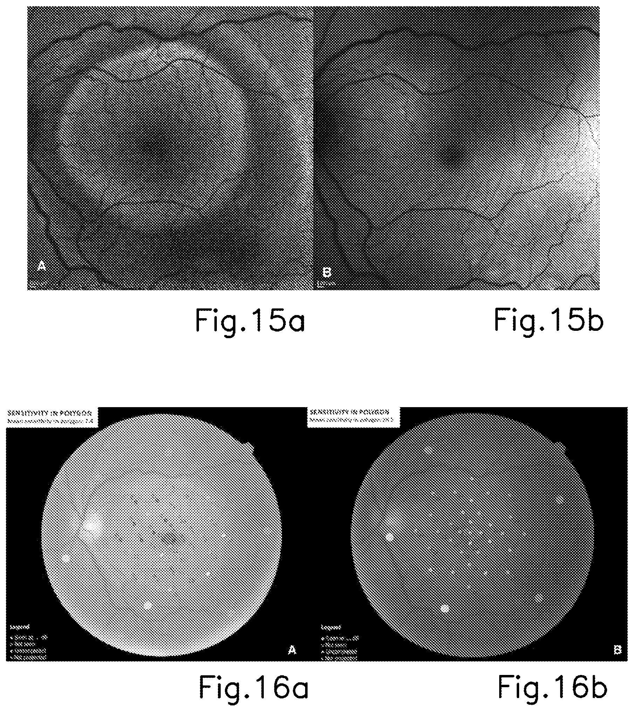

In addition, an image of the ocular fundus of the subject, obtained by autofluorescence examination (FAF Fundus Autofluorence) in a condition of reference, and an image of the ocular fundus of the subject three months after the aforementioned treatment are respectively shown in figures 15a and 15b. It can be observed that figure 15a shows a hyperautofluorescence ring at the periphery of the detachment of the pigment epithelium while figure 15b shows isoautofluorescence in the macular region without any legacy of retinal pigment epithelium atrophy.

Finally, figure 16a shows a reduction in retina sensitivity in the macular region with an average value of 7.4 dB. Microperimetry carried out three months after treatment (see figure 16b) shows an improvement in sensitivity in the macular region with an average value of 26.5 dB.

The reported cases back up the efficacy of the treatment administered by means of the device.

According to one aspect of the invention, in the case of a prophylaxis treatment, in particular for the prophylaxis of the dry age-related macular degeneration (dAMD), the aforementioned treatment steps, which constitute a treatment session, are performed by the computer program for at least one further treatment session, one week after the first session. It is preferable to repeat the treatment up to 4 sessions, at certain time intervals, preferably every week, to constitute a cycle of sessions.

Following the first cycle of sessions, the program also comprises the instruction to set a cycle of prophylactic maintenance treatment, by operating the LEDs 3, to be administered once in a quarter and which includes two treatment sessions to be carried out over a period of one week, 3-4 days apart. The cycle of prophylactic maintenance treatment should be carried out throughout the life of the patient.

It is preferable to perform a health check of the cells of the internal tissues of the eye at predetermined time distances, for example every 6-12 months from the end of the last treatment cycle.

According to a further aspect of the invention, in the case of treatment of severe forms of dry age-related macular degeneration (dAMD), the program executed by the electronic computer 41 selects the LEDs 3 arranged at the eye area and sets an initial treatment cycle. Each session of the initial treatment cycle comprises the aforementioned treatment steps but the time intervals are different, as indicated below.

Preferably the first time interval Δt₁ is greater than or equal to 10 minutes and the second time interval Δt₂ is less than 4 minutes. Even more preferably, the first time interval is equal to 12 minutes, or around ± 2 minutes with the duration of 12 minutes.

Even more preferably, the second time interval is equal to 2 minutes, or around ± 30 seconds with the duration of 2 minutes.

It is preferable to repeat the treatment session twice a week, for a maximum of 4 consecutive weeks, at the end of the initial 4-week treatment cycle.

Following the initial cycle of sessions, the program also comprises the instruction to set a maintenance treatment cycle, by operating the LEDs 3, which includes two sessions per week, for a period of three months from the end of the first month of the initial cycle. Each maintenance cycle session comprises the aforementioned treatment steps but the time intervals are reduced with respect to each treatment session of the initial cycle.

In particular, the first time interval Δt₁ is greater than 5 minutes and the second time interval Δt₂ is less than 1 minute. Even more preferably, the first time interval is equal to 5 minutes, or around ± 100 seconds with the duration of 5 minutes. Even more preferably, the second time interval is equal to 1 minute, or around ± 50 seconds with the duration of 1 minute.

Preferably, the program comprises the instruction to set a maintenance cycle repetition, by operating the LEDs, after a period comprised between 6 and 9 months from the initial cycle. The number of sessions for this second maintenance cycle is preferably comprised between 4 and 7, preferably it is equal to 6 sessions.

It is preferable to perform a check of the health status of the cells of the internal tissues of the eye at predetermined time distances, for example after 1 month, 4 months and 12 months from the end of the last treatment cycle.

According to a further aspect of the invention, in the case of treatment of non-neovascular age-related macular degeneration (nnAMD) and chronic central serous chorioretinopathy (cCSC), the program executed by the electronic computer 41 selects the LEDs 3 arranged at the eye area and sets an initial treatment cycle.

It is specified that central serous chorioretinopathy (CSC) is the fourth most common retinopathy that typically affects young and middle-aged adults, with a higher incidence in men. It is characterized by choroidal vascular abnormalities associated with a dysfunction of the retinal pigment epithelium (RPE), leading to the development of a detachment of the pigment epithelium (PED) and a serous neurosensory retinal detachment at the posterior pole. It is generally a self-limiting disease, although in 5-10% of cases the subretinal fluid (SRF) may persist for more than 6 months. It is generally a self-limiting disease, although in 5-10% of cases the subretinal fluid (SRF) may persist for more than 6 months, resulting in a chronic disease associated with damage to the RPE and/or photoreceptors.

The initial cycle includes one treatment session per week for 4 weeks. Each session of the initial treatment cycle comprises the aforementioned treatment steps. Following the first cycle of sessions, the program also comprises the instruction to set a maintenance treatment cycle, by operating the LEDs 3, which includes two sessions per week for a period of three months following the first initial month of treatment.

Preferably, the program comprises the instruction to set a maintenance cycle repetition, by operating said LEDs, from the fifth month to the twelfth month from the first session, for a total of a predetermined number of sessions, preferably comprised between 1 and 2 every 4 weeks.

It is preferable to perform a check of the health status of the cells of the internal tissues of the eye at predetermined time distances, for example after 1 month, 4 months and 12 months from the end of the last treatment cycle.

In the event that a mixed treatment is carried out, for example a treatment of the eye area together with a therapeutic treatment of other areas of the face, the further matrices 31 of LEDs 3 arranged at the affected regions are also activated.

In the case of mixed treatment, the operator proceeds, through the interface 6 of the control and/or power supply unit 4, with selecting the further LEDs 3 of interest and with entering the values of the parameters suitable for the type of treatment, for example the duration and the emission power.

The device described by way of example is susceptible to numerous modifications and variations according to different needs.

In the practical implementation of the invention, the materials employed, as well as the shape and sizes, may be any according to requirements.

Where technical features mentioned in each claim are followed by reference marks, such reference marks have been included for the sole purpose of increasing the understanding of the claims and consequently they do not have any limiting value on the scope of each element identified by way of example by such reference marks.

## Claims

1. A device for the treatment of ocular disorders, in particular for the treatment of the internal tissues of a user's eye, comprising
a mask (2);
a plurality of light-emitting diodes (3) distributed on the internal surface of said mask (2), said plurality of light-emitting diodes (3) comprising at least one matrix (30) of light-emitting diodes (3) arranged in areas of said internal surface of the mask (2) adapted to be facing, in use, the ocular areas of the user, said light-emitting diodes (3) being arranged substantially at the same distance from the eyes of the user, and configured to emit electromagnetic radiations adapted to stimulate the cellular function of the retinal cells; support means adapted to keep, in use, said mask (2) in a predetermined position in front of the user's face;
an external control and/or power supply unit (4) electrically connected to said light-emitting diodes (3) and provided with a control interface (6);
**characterized in that** said control and/or power supply unit (4) comprises an electronic computer (41); a memory (42) readable by said electronic computer, comprising instructions which, when executed by said electronic computer (41), cause said electronic computer (41) to perform the following steps:
a) receiving from an operator, through said control interface (6), commands relating to a protocol for treating the internal tissues of the user's eye;
b) automatically selecting a plurality of said light-emitting diodes (3) and automatically setting the wavelength and emission duration thereof (Δt);
c) instructing the user to close the eyes by means of a warning signal;
d) operating said plurality of said light-emitting diodes (3) for a first time interval Δt₁, in continuous mode, at a wavelength in an interval of ± 40 nm around 590 nm to emit electromagnetic radiations in the direction of the closed eyes of said user;
e) instructing said user to open the eyes by means of a warning signal;
f) operating said plurality of said light-emitting diodes (3) for a second time interval Δt₂, in pulsed mode, at a wavelength in an interval of ± 40 nm around 590 nm to emit electromagnetic radiations in the direction of the open eyes of said user;
g) instructing the user to close the eyes by means of a warning signal;
h) operating said plurality of said light-emitting diodes (3) for said first time interval Δt₁, in continuous mode, at a wavelength in an interval of ± 40 nm around 630 nm to emit electromagnetic radiations in the direction of a user's closed eyes;
i) instructing the user to open the eyes by means of a warning signal;
j) operating said plurality of said light-emitting diodes (3) for said second time interval Δt₂, in pulsed mode, at a wavelength in an interval of ± 40 nm around 630 nm to emit electromagnetic radiations in the direction of the open eyes of said user.

2. The device of claim 1, wherein said warning signal is an acoustic signal and/or a vibration signal and/or a signal displayed on said control interface (6).

3. The device of claim 2, wherein said signal displayed on said control interface (6) is an image and/or a text message.

4. The device of claim 1, 2 or 3, wherein said light-emitting diodes (3) are configured to emit a beam of electromagnetic radiations at a wavelength in an interval of ± 40 nm around 590 nm to inhibit the expression of the vascular endothelial growth factor (VEGF) and increase the expression of nitric oxide.

5. The device of any one of the preceding claims, wherein said light-emitting diodes (3) are configured to emit a beam of electromagnetic radiations at a wavelength in an interval of ± 40 nm around 630 nm to promote electron transfer and oxygen binding of cytochrome C oxidase (CCO) by increasing mitochondrial metabolic activity.

6. The device of any one of the preceding claims, wherein said memory (42) readable by said electronic computer comprises instructions which, when executed by said electronic computer (41), cause said electronic computer (41) to automatically select a plurality of said light-emitting diodes (3) and automatically set the wavelength and emission duration thereof to carry out a treatment of internal tissues of a user's eye.

7. The device of claim 6, wherein said memory (42) comprises instructions that, when executed by said electronic computer (41), cause said electronic computer (41), as a function of commands set by an operator through said control interface (6) relating to a protocol for treating the internal tissues of the user's eye, to automatically select a plurality of said light-emitting diodes (3) and automatically set the wavelength and emission duration thereof.

8. The device of any one of the preceding claims, wherein it comprises an image acquisition device adapted to acquire images of the eyes of a user and/or motion sensor means, said image acquisition device and/or said motion sensor means being adapted to detect whether the user has open or closed eyes.

9. The device of any one of the preceding claims, wherein it comprises an acoustic signal transmitter.

10. A computer program comprising instructions that cause the device according to any one of the preceding claims to perform the following steps:
a) receiving from an operator, through said control interface (6), commands relating to a protocol for treating the internal tissues of the user's eye;
b) automatically selecting a plurality of said light-emitting diodes (3) and automatically setting the wavelength and emission duration thereof (Δt);
c) instructing the user to close the eyes by means of a warning signal;
d) operating said plurality of said light-emitting diodes (3) for a first time interval Δt₁, in continuous mode, at a wavelength in an interval of ± 40 nm around 590 nm to emit electromagnetic radiations in the direction of the closed eyes of said user;
e) instructing the user to open the eyes by means of a warning signal;
f) operating said plurality of said light-emitting diodes (3) for a second time interval Δt₂, in pulsed mode, at a wavelength in an interval of ± 40 nm around 590 nm to emit electromagnetic radiations in the direction of the open eyes of said user;
g) instructing the user to close the eyes by means of a warning signal;
h) operating said plurality of said light-emitting diodes (3) for said first time interval Δt₁, in continuous mode, at a wavelength in an interval of ± 40 nm around 630 nm to emit electromagnetic radiations in the direction of a user's closed eyes;
i) instructing the user to open the eyes by means of a warning signal;
j) operating said plurality of said light-emitting diodes (3) for said second time interval Δt₂, in pulsed mode, at a wavelength in an interval of ± 40 nm around 630 nm to emit electromagnetic radiations in the direction of the open eyes of said user.

11. The computer program of claim 10, wherein said warning signal is an acoustic signal and/or a vibration signal and/or a signal displayed on said control interface (6).

12. The computer program of claim 11, wherein said signal displayed on said control interface (6) is an image and/or a text message.

13. The computer program of claim 10, 11 or 12, wherein before steps d, f and h it provides for the step of checking whether the eyes of said user are closed or open.

14. The computer program of claim 13, wherein said step of checking whether the eyes of said user are closed or open is performed by means of an image acquisition device, adapted to acquire at least one image of the eyes of the user, and/or by motion sensor means.

15. The computer program of any one of claims 10-14, wherein said first time interval Δt₁ is equal to 6 minutes, or around ± 1 minute with the duration of 6 minutes and said second time interval Δt2 is equal to 1 minute, or around ± 15 seconds with the duration of 1 minute.

16. The computer program of claim 15, wherein it comprises the further instruction to set a repetition of said steps a-j constituting a treatment session, by means of said device, for at least one further treatment session, preferably for further 6-7 said sessions, thus defining a treatment cycle, and to set a time interval between a session and a said further session comprised between 3 and 4 days.

17. The computer program of claim 16, wherein it comprises the further instruction to set a repetition of said treatment cycle, by operating said light-emitting diodes (3), after a period of time comprised between 6 and 9 months from the first treatment session, and to set a total number of treatment sessions comprised between 4 and 7 or equal to 6 sessions.

18. A computer-readable memory, into which the computer program according to claim 10 is loaded.

## Patentansprüche

1. Vorrichtung zur Behandlung von Augenerkrankungen, insbesondere zur Behandlung der inneren Gewebe des Auges eines Benutzers, umfassend eine Maske (2);
eine Mehrzahl von Leuchtdioden (3), die auf der Innenfläche der Maske (2) verteilt sind, wobei die Mehrzahl von Leuchtdioden (3) mindestens eine Matrix (30) von Leuchtdioden (3) umfasst, die in Bereichen der Innenfläche der Maske (2) angeordnet sind, welche dazu ausgebildet sind, im Gebrauch den Augenbereichen des Benutzers zugewandt zu sein, wobei die Leuchtdioden (3) im Wesentlichen im gleichen Abstand von den Augen des Benutzers angeordnet und dazu konfiguriert sind, elektromagnetische Strahlungen auszusenden, die geeignet sind, die Zellfunktion der Netzhautzellen zu stimulieren;
Trägermittel, die dazu ausgebildet sind, im Gebrauch die Maske (2) in einer vorbestimmten Position vor dem Gesicht des Benutzers zu halten;
eine externe Steuer- und/oder Versorgungseinheit (4), die mit den Leuchtdioden (3) elektrisch verbunden und mit einer Steuerschnittstelle (6) versehen ist;
**dadurch gekennzeichnet, dass** die Steuer- und/oder Versorgungseinheit (4) Folgendes umfasst: einen elektronischen Rechner (41); einen von dem elektronischen Rechner lesbaren Speicher (42), der Anweisungen umfasst, die bei ihrer Ausführung durch den elektronischen Rechner (41) den elektronischen Rechner (41) veranlassen, die folgenden Schritte auszuführen:
a) Empfangen von Befehlen, die ein Protokoll zur Behandlung der inneren Gewebe des Auges des Benutzers betreffen, von einem Bediener über die Steuerschnittstelle (6);
b) automatisches Auswählen einer Mehrzahl der Leuchtdioden (3) und automatisches Einstellen ihrer Wellenlänge und Emissionsdauer (Δt);
c) Auffordern des Benutzers mittels eines Warnsignals, die Augen zu schließen;
d) Betreiben der Mehrzahl der Leuchtdioden (3) während eines ersten Zeitintervalls Δt1 im Dauerbetrieb bei einer Wellenlänge in einem Intervall von ± 40 nm um 590 nm herum, um elektromagnetische Strahlungen in Richtung der geschlossenen Augen des Benutzers auszusenden;
e) Auffordern des Benutzers mittels eines Warnsignals, die Augen zu öffnen;
f) Betreiben der Mehrzahl der Leuchtdioden (3) während eines zweiten Zeitintervalls Δt2 im gepulsten Betrieb bei einer Wellenlänge in einem Intervall von ± 40 nm um 590 nm herum, um elektromagnetische Strahlungen in Richtung der geöffneten Augen des Benutzers auszusenden;
g) Auffordern des Benutzers mittels eines Warnsignals, die Augen zu schließen;
h) Betreiben der Mehrzahl der Leuchtdioden (3) während des ersten Zeitintervalls Δt1 im Dauerbetrieb bei einer Wellenlänge in einem Intervall von ± 40 nm um 630 nm herum, um elektromagnetische Strahlungen in Richtung der geschlossenen Augen eines Benutzers auszusenden;
i) Auffordern des Benutzers mittels eines Warnsignals, die Augen zu öffnen;
j) Betreiben der Mehrzahl der Leuchtdioden (3) während des zweiten Zeitintervalls Δt2 im gepulsten Betrieb bei einer Wellenlänge in einem Intervall von ± 40 nm um 630 nm herum, um elektromagnetische Strahlungen in Richtung der geöffneten Augen des Benutzers auszusenden.

2. Vorrichtung nach Anspruch 1, wobei das Warnsignal ein akustisches Signal und/oder ein Vibrationssignal und/oder ein auf der Steuerschnittstelle (6) angezeigtes Signal ist.

3. Vorrichtung nach Anspruch 2, wobei das auf der Steuerschnittstelle (6) angezeigte Signal ein Bild und/oder eine Textnachricht ist.

4. Vorrichtung nach Anspruch 1, 2 oder 3, wobei die Leuchtdioden (3) dazu konfiguriert sind, ein Bündel elektromagnetischer Strahlungen bei einer Wellenlänge in einem Intervall von ± 40 nm um 590 nm herum auszusenden, um die Expression des vaskulären endothelialen Wachstumsfaktors (VEGF) zu hemmen und die Expression von Stickstoffmonoxid zu erhöhen.

5. Vorrichtung nach einem der vorhergehenden Ansprüche, wobei die Leuchtdioden (3) dazu konfiguriert sind, ein Bündel elektromagnetischer Strahlungen bei einer Wellenlänge in einem Intervall von ± 40 nm um 630 nm herum auszusenden, um den Elektronentransfer und die Sauerstoffbindung der Cytochrom-c-Oxidase (CCO) durch Erhöhung der mitochondrialen Stoffwechselaktivität zu fördern.

6. Vorrichtung nach einem der vorhergehenden Ansprüche, wobei der von dem elektronischen Rechner lesbare Speicher (42) Anweisungen umfasst, die bei ihrer Ausführung durch den elektronischen Rechner (41) den elektronischen Rechner (41) veranlassen, automatisch eine Mehrzahl der Leuchtdioden (3) auszuwählen und automatisch deren Wellenlänge und Emissionsdauer einzustellen, um eine Behandlung der inneren Gewebe des Auges eines Benutzers durchzuführen.

7. Vorrichtung nach Anspruch 6, wobei der Speicher (42) Anweisungen umfasst, die bei ihrer Ausführung durch den elektronischen Rechner (41) den elektronischen Rechner (41) veranlassen, in Abhängigkeit von Befehlen, die von einem Bediener über die Steuerschnittstelle (6) eingestellt werden und ein Protokoll zur Behandlung der inneren Gewebe des Auges des Benutzers betreffen, automatisch eine Mehrzahl der Leuchtdioden (3) auszuwählen und automatisch deren Wellenlänge und Emissionsdauer einzustellen.

8. Vorrichtung nach einem der vorhergehenden Ansprüche, die eine Bilderfassungsvorrichtung, die dazu ausgebildet ist, Bilder der Augen eines Benutzers zu erfassen, und/oder Bewegungssensormittel umfasst, wobei die Bilderfassungsvorrichtung und/oder die Bewegungssensormittel dazu ausgebildet sind, festzustellen, ob der Benutzer die Augen geöffnet oder geschlossen hat.

9. Vorrichtung nach einem der vorhergehenden Ansprüche, die einen akustischen Signalgeber umfasst.

10. Computerprogramm, umfassend Anweisungen, die die Vorrichtung nach einem der vorhergehenden Ansprüche veranlassen, die folgenden Schritte auszuführen:
a) Empfangen von Befehlen, die ein Protokoll zur Behandlung der inneren Gewebe des Auges des Benutzers betreffen, von einem Bediener über die Steuerschnittstelle (6);
b) automatisches Auswählen einer Mehrzahl der Leuchtdioden (3) und automatisches Einstellen ihrer Wellenlänge und Emissionsdauer (Δt);
c) Auffordern des Benutzers mittels eines Warnsignals, die Augen zu schließen;
d) Betreiben der Mehrzahl der Leuchtdioden (3) während eines ersten Zeitintervalls Δt1 im Dauerbetrieb bei einer Wellenlänge in einem Intervall von ± 40 nm um 590 nm herum, um elektromagnetische Strahlungen in Richtung der geschlossenen Augen des Benutzers auszusenden;
e) Auffordern des Benutzers mittels eines Warnsignals, die Augen zu öffnen;
f) Betreiben der Mehrzahl der Leuchtdioden (3) während eines zweiten Zeitintervalls Δt2 im gepulsten Betrieb bei einer Wellenlänge in einem Intervall von ± 40 nm um 590 nm herum, um elektromagnetische Strahlungen in Richtung der geöffneten Augen des Benutzers auszusenden;
g) Auffordern des Benutzers mittels eines Warnsignals, die Augen zu schließen;
h) Betreiben der Mehrzahl der Leuchtdioden (3) während des ersten Zeitintervalls Δt1 im Dauerbetrieb bei einer Wellenlänge in einem Intervall von ± 40 nm um 630 nm herum, um elektromagnetische Strahlungen in Richtung der geschlossenen Augen eines Benutzers auszusenden;
i) Auffordern des Benutzers mittels eines Warnsignals, die Augen zu öffnen;
j) Betreiben der Mehrzahl der Leuchtdioden (3) während des zweiten Zeitintervalls Δt2 im gepulsten Betrieb bei einer Wellenlänge in einem Intervall von ± 40 nm um 630 nm herum, um elektromagnetische Strahlungen in Richtung der geöffneten Augen des Benutzers auszusenden.

11. Computerprogramm nach Anspruch 10, wobei das Warnsignal ein akustisches Signal und/oder ein Vibrationssignal und/oder ein auf der Steuerschnittstelle (6) angezeigtes Signal ist.

12. Computerprogramm nach Anspruch 11, wobei das auf der Steuerschnittstelle (6) angezeigte Signal ein Bild und/oder eine Textnachricht ist.

13. Computerprogramm nach Anspruch 10, 11 oder 12, das vor den Schritten d, f und h den Schritt des Prüfens, ob die Augen des Benutzers geschlossen oder geöffnet sind, vorsieht.

14. Computerprogramm nach Anspruch 13, wobei der Schritt des Prüfens, ob die Augen des Benutzers geschlossen oder geöffnet sind, mittels einer Bilderfassungsvorrichtung, die dazu ausgebildet ist, mindestens ein Bild der Augen des Benutzers zu erfassen, und/oder mittels Bewegungssensormitteln durchgeführt wird.

15. Computerprogramm nach einem der Ansprüche 10 bis 14, wobei das erste Zeitintervall Δt1 gleich 6 Minuten beträgt oder in einem Bereich von etwa ± 1 Minute um die Dauer von 6 Minuten liegt und das zweite Zeitintervall Δt2 gleich 1 Minute beträgt oder in einem Bereich von etwa ± 15 Sekunden um die Dauer von 1 Minute liegt.

16. Computerprogramm nach Anspruch 15, das die weitere Anweisung umfasst, mittels der Vorrichtung eine Wiederholung der Schritte a bis j, die eine Behandlungssitzung bilden, für mindestens eine weitere Behandlungssitzung, vorzugsweise für weitere 6 bis 7 solcher Sitzungen, einzustellen, wodurch ein Behandlungszyklus definiert wird, und ein Zeitintervall zwischen einer Sitzung und einer solchen weiteren Sitzung einzustellen, das zwischen 3 und 4 Tagen liegt.

17. Computerprogramm nach Anspruch 16, das die weitere Anweisung umfasst, eine Wiederholung des Behandlungszyklus durch Betreiben der Leuchtdioden (3) nach einem Zeitraum zwischen 6 und 9 Monaten ab der ersten Behandlungssitzung einzustellen und eine Gesamtzahl von Behandlungssitzungen zwischen 4 und 7 oder gleich 6 Sitzungen einzustellen.

18. Computerlesbarer Speicher, in den das Computerprogramm nach Anspruch 10 geladen ist.

## Revendications

1. Dispositif pour le traitement de troubles oculaires, en particulier pour le traitement des tissus internes de l'œil d'un utilisateur, comprenant un masque (2) ;
une pluralité de diodes électroluminescentes (3) réparties sur la surface interne dudit masque (2), ladite pluralité de diodes électroluminescentes (3) comprenant au moins une matrice (30) de diodes électroluminescentes (3) disposées dans des zones de ladite surface interne du masque (2) qui sont adaptées pour être tournées, en utilisation, vers les zones oculaires de l'utilisateur, lesdites diodes électroluminescentes (3) étant disposées sensiblement à la même distance des yeux de l'utilisateur, et configurées pour émettre des rayonnements électromagnétiques adaptés pour stimuler la fonction cellulaire des cellules rétiniennes ;
des moyens de support adaptés pour maintenir, en utilisation, ledit masque (2) dans une position prédéterminée devant le visage de l'utilisateur ;
une unité externe de commande et/ou d'alimentation (4) reliée électriquement auxdites diodes électroluminescentes (3) et pourvue d'une interface de commande (6) ;
**caractérisé en ce que** ladite unité de commande et/ou d'alimentation (4) comprend un calculateur électronique (41) ; une mémoire (42) lisible par ledit calculateur électronique, comprenant des instructions qui, lorsqu'elles sont exécutées par ledit calculateur électronique (41), amènent ledit calculateur électronique (41) à exécuter les étapes suivantes :
a) recevoir d'un opérateur, par l'intermédiaire de ladite interface de commande (6), des commandes relatives à un protocole de traitement des tissus internes de l'œil de l'utilisateur ;
b) sélectionner automatiquement une pluralité desdites diodes électroluminescentes (3) et régler automatiquement la longueur d'onde et la durée d'émission de celles-ci (Δt) ;
c) donner pour instruction à l'utilisateur, au moyen d'un signal d'avertissement, de fermer les yeux ;
d) faire fonctionner ladite pluralité desdites diodes électroluminescentes (3) pendant un premier intervalle de temps Δt1, en mode continu, à une longueur d'onde dans un intervalle de ± 40 nm autour de 590 nm afin d'émettre des rayonnements électromagnétiques en direction des yeux fermés dudit utilisateur ;
e) donner pour instruction audit utilisateur, au moyen d'un signal d'avertissement, d'ouvrir les yeux ;
f) faire fonctionner ladite pluralité desdites diodes électroluminescentes (3) pendant un deuxième intervalle de temps Δt2, en mode pulsé, à une longueur d'onde dans un intervalle de ± 40 nm autour de 590 nm afin d'émettre des rayonnements électromagnétiques en direction des yeux ouverts dudit utilisateur ;
g) donner pour instruction à l'utilisateur, au moyen d'un signal d'avertissement, de fermer les yeux ;
h) faire fonctionner ladite pluralité desdites diodes électroluminescentes (3) pendant ledit premier intervalle de temps Δt1, en mode continu, à une longueur d'onde dans un intervalle de ± 40 nm autour de 630 nm afin d'émettre des rayonnements électromagnétiques en direction des yeux fermés d'un utilisateur ;
i) donner pour instruction à l'utilisateur, au moyen d'un signal d'avertissement, d'ouvrir les yeux ;
j) faire fonctionner ladite pluralité desdites diodes électroluminescentes (3) pendant ledit deuxième intervalle de temps Δt2, en mode pulsé, à une longueur d'onde dans un intervalle de ± 40 nm autour de 630 nm afin d'émettre des rayonnements électromagnétiques en direction des yeux ouverts dudit utilisateur.

2. Dispositif selon la revendication 1, dans lequel ledit signal d'avertissement est un signal acoustique et/ou un signal vibratoire et/ou un signal affiché sur ladite interface de commande (6).

3. Dispositif selon la revendication 2, dans lequel ledit signal affiché sur ladite interface de commande (6) est une image et/ou un message textuel.

4. Dispositif selon l'une quelconque des revendications 1, 2 ou 3, dans lequel lesdites diodes électroluminescentes (3) sont configurées pour émettre un faisceau de rayonnements électromagnétiques à une longueur d'onde dans un intervalle de ± 40 nm autour de 590 nm afin d'inhiber l'expression du facteur de croissance de l'endothélium vasculaire (VEGF) et d'augmenter l'expression du monoxyde d'azote.

5. Dispositif selon l'une quelconque des revendications précédentes, dans lequel lesdites diodes électroluminescentes (3) sont configurées pour émettre un faisceau de rayonnements électromagnétiques à une longueur d'onde dans un intervalle de ± 40 nm autour de 630 nm afin de favoriser le transfert d'électrons et la fixation de l'oxygène par la cytochrome c oxydase (CCO) en augmentant l'activité métabolique mitochondriale.

6. Dispositif selon l'une quelconque des revendications précédentes, dans lequel ladite mémoire (42) lisible par ledit calculateur électronique comprend des instructions qui, lorsqu'elles sont exécutées par ledit calculateur électronique (41), amènent ledit calculateur électronique (41) à sélectionner automatiquement une pluralité desdites diodes électroluminescentes (3) et à régler automatiquement la longueur d'onde et la durée d'émission de celles-ci afin de réaliser un traitement des tissus internes de l'œil d'un utilisateur.

7. Dispositif selon la revendication 6, dans lequel ladite mémoire (42) comprend des instructions qui, lorsqu'elles sont exécutées par ledit calculateur électronique (41), amènent ledit calculateur électronique (41), en fonction de commandes réglées par un opérateur au moyen de ladite interface de commande (6) et relatives à un protocole de traitement des tissus internes de l'œil de l'utilisateur, à sélectionner automatiquement une pluralité desdites diodes électroluminescentes (3) et à régler automatiquement la longueur d'onde et la durée d'émission de celles-ci.

8. Dispositif selon l'une quelconque des revendications précédentes, **caractérisé en ce qu'**il comprend un dispositif d'acquisition d'images adapté pour acquérir des images des yeux d'un utilisateur et/ou des moyens formant capteur de mouvement, ledit dispositif d'acquisition d'images et/ou lesdits moyens formant capteur de mouvement étant adaptés pour détecter si l'utilisateur a les yeux ouverts ou fermés.

9. Dispositif selon l'une quelconque des revendications précédentes, **caractérisé en ce qu'**il comprend un émetteur de signal acoustique.

10. Programme d'ordinateur comprenant des instructions qui amènent le dispositif selon l'une quelconque des revendications précédentes à exécuter les étapes suivantes :
a) recevoir d'un opérateur, par l'intermédiaire de ladite interface de commande (6), des commandes relatives à un protocole de traitement des tissus internes de l'œil de l'utilisateur ;
b) sélectionner automatiquement une pluralité desdites diodes électroluminescentes (3) et régler automatiquement la longueur d'onde et la durée d'émission de celles-ci (Δt) ;
c) donner pour instruction à l'utilisateur, au moyen d'un signal d'avertissement, de fermer les yeux ;
d) faire fonctionner ladite pluralité desdites diodes électroluminescentes (3) pendant un premier intervalle de temps Δt1, en mode continu, à une longueur d'onde dans un intervalle de ± 40 nm autour de 590 nm afin d'émettre des rayonnements électromagnétiques en direction des yeux fermés dudit utilisateur ;
e) donner pour instruction audit utilisateur, au moyen d'un signal d'avertissement, d'ouvrir les yeux ;
f) faire fonctionner ladite pluralité desdites diodes électroluminescentes (3) pendant un deuxième intervalle de temps Δt2, en mode pulsé, à une longueur d'onde dans un intervalle de ± 40 nm autour de 590 nm afin d'émettre des rayonnements électromagnétiques en direction des yeux ouverts dudit utilisateur ;
g) donner pour instruction à l'utilisateur, au moyen d'un signal d'avertissement, de fermer les yeux ;
h) faire fonctionner ladite pluralité desdites diodes électroluminescentes (3) pendant ledit premier intervalle de temps Δt1, en mode continu, à une longueur d'onde dans un intervalle de ± 40 nm autour de 630 nm afin d'émettre des rayonnements électromagnétiques en direction des yeux fermés d'un utilisateur ;
i) donner pour instruction à l'utilisateur, au moyen d'un signal d'avertissement, d'ouvrir les yeux ;
j) faire fonctionner ladite pluralité desdites diodes électroluminescentes (3) pendant ledit deuxième intervalle de temps Δt2, en mode pulsé, à une longueur d'onde dans un intervalle de ± 40 nm autour de 630 nm afin d'émettre des rayonnements électromagnétiques en direction des yeux ouverts dudit utilisateur.

11. Programme d'ordinateur selon la revendication 10, dans lequel ledit signal d'avertissement est un signal acoustique et/ou un signal vibratoire et/ou un signal affiché sur ladite interface de commande (6).

12. Programme d'ordinateur selon la revendication 11, dans lequel ledit signal affiché sur ladite interface de commande (6) est une image et/ou un message textuel.

13. Programme d'ordinateur selon l'une quelconque des revendications 10, 11 ou 12, **caractérisé en ce qu'**il prévoit, avant les étapes d, f et h, l'étape consistant à vérifier si les yeux dudit utilisateur sont fermés ou ouverts.

14. Programme d'ordinateur selon la revendication 13, dans lequel ladite étape consistant à vérifier si les yeux dudit utilisateur sont fermés ou ouverts est réalisée au moyen d'un dispositif d'acquisition d'images, adapté pour acquérir au moins une image des yeux de l'utilisateur, et/ou par des moyens formant capteur de mouvement.

15. Programme d'ordinateur selon l'une quelconque des revendications 10 à 14, dans lequel ledit premier intervalle de temps Δt1 est égal à 6 minutes, ou à environ ± 1 minute autour de la durée de 6 minutes, et ledit deuxième intervalle de temps Δt2 est égal à 1 minute, ou à environ ± 15 secondes autour de la durée d'une minute.

16. Programme d'ordinateur selon la revendication 15, **caractérisé en ce qu'**il comprend l'instruction supplémentaire de régler une répétition desdites étapes a à j constituant une séance de traitement, au moyen dudit dispositif, pour au moins une autre séance de traitement, de préférence pour 6 à 7 autres desdites séances, définissant ainsi un cycle de traitement, et de régler, entre une séance et une autre desdites séances, un intervalle de temps compris entre 3 et 4 jours.

17. Programme d'ordinateur selon la revendication 16, **caractérisé en ce qu'**il comprend l'instruction supplémentaire de régler une répétition dudit cycle de traitement, en faisant fonctionner lesdites diodes électroluminescentes (3), après une période de temps comprise entre 6 et 9 mois à compter de la première séance de traitement, et de régler un nombre total de séances de traitement compris entre 4 et 7 ou égal à 6 séances.

18. Mémoire lisible par ordinateur, dans laquelle est chargé le programme d'ordinateur selon la revendication 10.
